# EUROPÄISCHE PATENTSCHRIFT

(11) **EP 2 790 784 B1**
(45) Veröffentlichungstag und Bekanntmachung des Hinweises auf die Patenterteilung: **22.06.2022**
(21) Anmeldenummer: 12787411.3
(22) Anmeldetag: 05.11.2012
(51) Int. Cl.: A61N 5/10

(54) **VERFAHREN UND VORRICHTUNG ZUR ERSTELLUNG EINES BESTRAHLUNGSPLANS BEI BEWEGTEM ZIELVOLUMEN OHNE BEWEGUNGSKOMPENSATION**
METHOD AND APPARATUS FOR PREPARING AN IRRADIATION PLAN FOR A MOVING TARGET VOLUME WITHOUT A MOVEMENT COMPENSATION
PROCÉDÉ ET APPAREIL PERMETTANT D'ÉTABLIR UN PLAN D'EXPOSITION À UN RAYONNEMENT POUR UN VOLUME CIBLE SE DÉPLAÇANT, SANS COMPENSATION DU DÉPLACEMENT

(30) Priorität: 13.12.2011 DE 102011056339
(43) Veröffentlichungstag der Anmeldung: 22.10.2014
(73) Patentinhaber: GSI Helmholtzzentrum für Schwerionenforschung GmbH, 64291 Darmstadt (DE)
(72) Erfinder: BERT, Christoph, 91080 Uttenreuth (DE); GRAEFF, Christian, 64331 Weiterstadt (DE)
(86) Internationale Anmeldenummer: PCT/EP2012/071853
(87) Internationale Veröffentlichungsnummer: WO 2013/087297

(56) Entgegenhaltungen:
- WO-A1-2005/120641
- WO-A2-2011/005329
- DE-A1-102007 045 879
- DE-B4-102007 045 879
- US-A1- 2006 074 292
- US-A1- 2008 021 300

## Beschreibung

Die Erfindung betrifft ein Verfahren zur Erstellung eines Bestrahlungsplans zur Bestrahlung eines zumindest bereichsweise bewegten Zielvolumenbereichs in einem Objekt mit einem vorzugsweise bewegten Teilchenstrahl, bei dem mit einer Abbildungsfunktion für eine Mehrzahl an Bewegungszuständen der jeweilige, durch Beaufschlagung eines Bestrahlungspunkts mittels des vorzugsweise bewegten Teilchenstrahls bewirkte Dosiseintrag in zumindest einem Zielpunktvolumen, wenn ein Bestrahlungspunkt angefahren wird, bestimmt wird. Die Erfindung betrifft darüber hinaus eine geeignete Vorrichtung zur Durchführung eines solchen Verfahrens.

Die Verwendung von Strahlung unterschiedlichster Art und Energie zur Beaufschlagung, Bearbeitung von zu bearbeitenden Werkstücken bzw. der Veränderung von Materialeigenschaften von zu bearbeitenden Werkstücken hat sich zwischenzeitlich im Stand der Technik für unterschiedlichste Anwendungsgebiete durchgesetzt.

Als Strahlungsarten kommen dabei nicht nur Photonenstrahlung (also insbesondere Lichtbeaufschlagung, Beaufschlagung mit Röntgenstrahlung, UV-Licht, Infrarotlicht und dergleichen), sondern auch insbesondere Partikelstrahlung in Betracht. Die Partikel können dabei im Wesentlichen beliebig sein (wobei unter Partikeln in diesem Zusammenhang insbesondere Partikel zu verstehen sind, welche eine - wenn auch gegebenenfalls äußerst kleine - Ruhemasse aufweisen). Rein beispielhaft sind Hadronen und Leptonen zu nennen, insbesondere auch Neutrinos, Elektronen, Positronen, Pionen, Mesonen, Protonen, Neutronen, Atomkerne (beispielsweise He-Kerne), Atome bzw. Moleküle sowie Ionen (insbesondere auch Schwerionen wie Sauerstoffionen, Heliumionen, Neonionen oder Kohlenstoffionen).

Dabei ist allen Bestrahlungsarten gemeinsam, dass mit der Strahlung in dem mit der Strahlung beaufschlagten Gegenstand eine bestimmte Energie deponiert wird. Die Art und Weise, wie diese Energie deponiert wird unterscheidet sich jedoch zum Teil stark. Während beispielsweise bei Photonenstrahlung der Energieverlust in weiten Energiebereichen annähernd linear zur durchdrungenen Materie ist, weisen Teilchenstrahlen, hier insbesondere Hadronen-Teilchen (speziell Protonen, Ionen und Schwerionen), einen ausgeprägten, so genannten Bragg-Peak auf. Die Teilchen verlieren also beim Durchdringen von Materie zunächst vergleichsweise wenig Energie auf ihrem Weg. Kurz bevor die Teilchen "stecken bleiben" wird jedoch der größte Teil der Energie in die mit der Strahlung beaufschlagte Materie abgegeben. Durch diesen Bragg-Peak können nicht nur zweidimensional strukturierte Dosisbeaufschlagungen realisiert werden, sondern insbesondere auch dreidimensional strukturierte Dosisbeaufschlagungen (also unterschiedliche deponierte Strahlungsdosen in unterschiedlichen Tiefen des bestrahlten Objekts).

Nicht nur die Art der verwendeten Strahlung ist mannigfaltig, sondern auch die Art der mit Strahlung beaufschlagten Objekte. Um lediglich einige technische Anwendungsgebiete zu nennen, ist beispielsweise im Zusammenhang mit der Beaufschlagung mit Photonen an Strukturierungsverfahren mit Masken und Materieabtrag bzw. Materieauftrag bei der Herstellung von strukturierten Halbleiterbauteilen (beispielsweise Speicherbausteine, Mikroprozessoren und dergleichen) zu denken. Photonen können auch zum Schneiden und/oder zum Verschweißen von Werkstücken verwendet werden (insbesondere dann, wenn die Photonenstrahlung in Form eines hochenergetischen Laserstrahls vorliegt).

Ein Anwendungsbeispiel für Elektronenstrahlen ist das so genannte Elektronenstrahlschweißen, mit dem beispielsweise zwei metallische Werkstücke miteinander verschweißt werden können. Selbstverständlich sind auch Trenn- bzw. Strukturierungsvorgänge denkbar.

In der Medizin bzw. der Veterinärmedizin findet Strahlung zu therapeutischen Zwecken Verwendung. Bekannt ist beispielsweise Röntgenstrahlung zur Erstellung von Röntgenbildern (einschließlich dreidimensionaler Bilder durch so genannte CT-Verfahren; CT für Computertomographie). Auch Elektronenstrahlen werden bereits seit einigen Jahrzehnten in der Medizin verwendet, beispielsweise zur Therapie von Krebstumoren. Zwischenzeitlich hat sich auch die Therapie von Tumoren mit Protonen und Ionen (insbesondere Schwerionen) als feste Größe in der Medizin etabliert. Aufgrund des bereits beschriebenen Bragg-Peaks bei Protonen/lonen/Schwerionen ist es möglich, durch entsprechende Ansteuerung eines Teilchenstrahls (beispielsweise im Rahmen eines Scanning-Vorgangs) ein dreidimensional abgegrenztes und strukturiertes Gebiet (also insbesondere einen Tumor) in einem Patienten gezielt mit Strahlung zu beaufschlagen, während das umliegende Gewebe weitestgehend geschont wird. Zwischenzeitlich sind Genauigkeiten im Millimeter-Bereich realisierbar. Hierbei werden zum Teil aufgeweitete Teilchenstrahle verwendet, die beispielsweise durch Paraffinplatten mit unterschiedlicher Dicke hinsichtlich ihrer Energie (und damit ihrer Eindringtiefe in das Gewebe) ortsaufgelöst strukturiert werden. In der Regel werden zwischenzeitlich jedoch meist sogenannte Scan-Verfahren genutzt, bei denen ein üblicherweise dünner (bleistiftdünner) Teilchenstrahl über geeignete Ablenkmagneten und eine geeignete Energievariation (bewirkt eine Variation hinsichtlich der Eindringtiefe) sukzessive die unterschiedlichen, mit einer Dosis zu beaufschlagendem Volumenbereiche des zu bestrahlenden Objekts "anfährt".

Um eine Behandlung von dreidimensionalen Volumenbereichen in einem Körper (also insbesondere eine Krebstherapie in einem Patienten) durchführen zu können, ist üblicherweise die Erstellung eines so genannten Bestrahlungsplans erforderlich. Hierbei wird ein bestimmtes Bestrahlungsmuster simuliert (also ein Ablauf mit unterschiedlichen x-y-Ablenkungen des Teilchenstrahls sowie geeigneten Energievariationen des Teilchenstrahls) und dabei der hierdurch jeweils verursachte Dosiseintrag in den mit der Strahlung beaufschlagten Körper ortsabhängig berechnet. Denn obgleich die deponierte Dosis im bestrahlten Objekt auf den Bereich des Bragg-Peaks konzentriert ist, so wird dennoch, insbesondere in proximal zum Bestrahlungspunkt längs des Teilchenstrahls liegenden Bereichen eine bestimmte Dosis deponiert. Im Rahmen der Bestrahlungsplanung wird nun versucht einen Bestrahlungsplan zu erstellen, mit dem innerhalb eines erwünschten, zu bestrahlenden Zielvolumenbereichs eine gewisse Mindestdosis sicher überschritten wird (so dass beispielsweise Krebszellen mit Sicherheit zerstört werden), umgebendes Gewebe jedoch möglichst geschont wird, also eine gewisse maximale Dosis nicht überschritten wird. Die hierbei anzusetzende maximale Dosis kann dabei stark von dem durchstrahlten Objektbereich variieren. Wenn beispielsweise bei medizinischen Anwendungen besonders lebenswichtige und/oder strahlungsempfindliche Gewebebereiche vorhanden sind, so müssen diese maximal geschont werden. Man spricht in diesem Zusammenhang in der Regel von OARs (OAR für Organ at Risk). Ein für den jeweiligen Einsatz besonders geeigneter Bestrahlungsplan kann dabei durch unterschiedliche Ansteuerung des Teilchenstrahls und/oder durch Einbringen des Teilchenstrahls aus mehreren unterschiedlichen Richtungen (gegebenenfalls auch eine Vielzahl von unterschiedlichen Richtungen, einschließlich eines zumindest zeitweise kontinuierlichen Verschwenkens) erfolgen.

Besondere Probleme treten auf, wenn sich (Teilbereiche des) zu bestrahlenden Objekts bewegen. Eine Bewegung kann dabei nicht nur Translationsbewegungen, sondern auch Torsionsbewegungen und/oder Stauchungen bzw. Dehnungen umfassen. Besonders in Kombination mit Scan-Verfahren können hier die Bewegungen des Objekts und des Teilchenstrahls miteinander "interferieren" und zu vergleichsweise schlechten Bestrahlungsergebnissen führen, wenn nicht geeignete Gegenmaßnahmen ergriffen werden.

Um bewegte Zielgebiete bestrahlen zu können, wurden im Stand der Technik unterschiedlichste Lösungsansätze vorgeschlagen.

Ein erster Lösungsansatz besteht beispielsweise darin, durch eine entsprechende Nachführung des Teilchenstrahls (so genanntes "Tracking") die Bewegung des Zielvolumenbereichs im Objekt zu kompensieren. Problematisch sind bei diesem Verfahren jedoch der stark erhöhte apparative Aufwand sowie das Problem, dass im Rahmen der Vorab-Bestrahlungsplanung Dosisdepositionseffekte in Objektbereichen außerhalb des aktiv bestrahlten Bestrahlungspunkts nicht vorhergesagt werden können. Dadurch werden bestimmte, vergleichsweise aufwändige Überprüfungs- und Kompensationsverfahren erforderlich. Auch die Feststellung der tatsächlichen Bewegung des Zielgebiets im Rahmen der eigentlichen Bestrahlung erweist sich oftmals als problematisch oder praktisch nicht möglich. Insbesondere in der Medizin sind auch gewisse Bewegungsarten einem derartigen "Tracking" nicht bzw. nur schwer zugänglich.

Die Deutsche Offenlegungsschrift DE 102007045879A1 beschreibt ein Verfahren zum Erstellen eines Bestrahlungsplans mit Korrekturwerten, die basierend auf Transformationsparametern bestimmt werden, wobei die Transformationsparameter die geometrischen Positionen des Bewegungszustands, der zur Erstellung des Bestrahlungsplans verwendet wurde, in die entsprechenden geometrischen Positionen weiterer Bewegungszustände überführen und genutzt werden, um während der Bestrahlung den Bestrahlungsplan an den jeweiligen Bewegungszustand anzupassen.

Ein anderer Vorschlag besteht darin, dass bei der Bestrahlungsplanung sämtliche möglichen Bewegungszustände vorab bestimmt werden (beispielsweise durch CT-Verfahren), und das im Rahmen der Bestrahlung bestrahlte Gebiet ausreichend groß und geeignet gewählt wird, so dass der zu bestrahlende Bereich in jedem Fall mit einer gewissen Mindestdosis beaufschlagt wird. Hierbei wird eine gewisse (an sich unerwünschte) Schädigung von umliegendem Gewebe bewusst in Kauf genommen (bzw. sogar im Rahmen von so genannten "Sicherheitssäumen" bewusst in die Planung mit einbezogen). Problematisch ist bei der Planung, dass insbesondere bei medizinischen Anwendungen eine (innere) Bewegung eines Zielvolumenbereichs im Patienten nicht nur eine Veränderung der geometrischen Lage des entsprechenden Volumenbereichs zur Folge hat, sondern insbesondere über Dehnungs- und Stauchungseffekte und die damit einhergehenden Dichteänderungen einen zusätzlichen Effekt auf die Durchdringungslänge des Teilchenstrahls im Gewebe hat.

Ein Verfahren zur Berücksichtigung derartiger Effekte wurde beispielsweise in der Deutschen Offenlegungsschrift DE 10 2007 014 723 A1 beschrieben. Hierbei wird ein Planungszielvolumen bestimmt, indem zunächst ein zu dem Mindestzielvolumen in dem Körper äquivalentes Zielvolumen in einem fiktiven homogenen Körper bestimmt wird. Das äquivalente Zielvolumen wird um einen Sicherheitssaum erweitert, um das Planungszielvolumen zu bestimmen. Problematisch bei dem dort vorgeschlagenen Verfahren ist, dass die Umrechnung in einen fiktiven homogenen Körper stark von der Einfallsrichtung des Teilchenstrahls abhängt. Eine Erstellung/Optimierung eines Bestrahlungsplans, bei dem ein Strahleintrag aus zwei oder mehr unterschiedlichen Richtungen erfolgt, ist mit dem dort vorgeschlagenen Verfahren nicht möglich. Derartige Verfahren mit unterschiedlichen Strahleintragsrichtungen sind jedoch erwünscht, um die Belastung von umgebendem Gewebe (insbesondere von OAR-Gewebe) nach Möglichkeit zu verringern.

Auch wenn bereits bestimmte Verbesserungen des dort beschriebenen Verfahrens durch geeignete Transformations- und Rücktransformationsalgorithmen angedacht wurden, weisen bekannte Verfahren nach wie vor Probleme auf. Insbesondere dann, wenn eine Bewegung des Zielvolumenbereichs in Kombination mit unterschiedlichen Einstrahlrichtungen kombiniert wird, kann es bei bekannten Verfahren lokal zu Abweichungen von einer zu deponierenden Soll-Dosis (so genannte Fehldosierung) kommen. Vor allem "Lücken" (lokale Unterdosierungen) im bestrahlten Gebiet sind jedoch höchst unerwünscht, da dadurch Krebszellen überleben können und dadurch der Therapieerfolg massiv gefährdet ist.

Es besteht somit nach wie vor ein Bedarf an einem Verfahren zur Erstellung eines Bestrahlungsplans, welches gegenüber im Stand der Technik bekannter Verfahren verbessert ist. Ebenso besteht ein Bedarf an Vorrichtungen zur Erstellung eines Bestrahlungsplans, welche gegenüber im Stand der Technik bekannten Vorrichtungen verbessert sind.

Die vorliegende Erfindung löst diese Aufgaben.

Es wird ein Verfahren zur Erstellung eines Bestrahlungsplans zur Bestrahlung eines zumindest bereichsweise bewegten Zielvolumenbereichs in einem Objekt mit einem Teilchenstrahl gemäß Anspruch 1 vorgeschlagen.

Es wird vorgeschlagen, ein Verfahren zur Erstellung eines Bestrahlungsplans zur Bestrahlung eines zumindest bereichsweise bewegten Zielvolumenbereichs in einem Objekt mit einem Teilchenstrahl, bei dem mit einer Abbildungsfunktion der jeweilige, durch Beaufschlagung eines Bestrahlungspunkts mittels des vorzugsweise bewegten Teilchenstrahls bewirkte Dosiseintrag in zumindest einem Zielpunktvolumen bestimmt wird, dahingehend weiterzubilden, dass die Abbildungsfunktion zusätzlich von einer Mehrzahl an Bewegungszuständen des bewegten Zielvolumenbereichs abhängt. Bei dem Bestrahlungsplan handelt es sich um einen Satz von unterschiedlichen Ansteuerungsparametern für den Teilchenstrahl. Als Teilchenstrahl bieten sich insbesondere hadronische und/oder leptonische Partikel an, insbesondere Protonen, Heliumionen, Neonionen, und/oder sonstige Ionen, vorzugsweise Schwerionen, an. Der Teilchenstrahl ist vorzugsweise ein bewegter Teilchenstrahl. Der Bestrahlungsplan kann (speziell im Falle eines bewegten Teilchenstrahls) insbesondere als eine Art zeitlicher Funktion eines wandernden Bestrahlungspunkts aufgefasst werden. Unter einem Bestrahlungspunkt kann also insbesondere ein einzelner Satz an Parametern für den Teilchenstrahl verstanden werden (insbesondere ein Zahlentupel). Insbesondere kommen als Parameter die Energie der Teilchen im Teilchenstrahl, die Teilchensorte, die Ionisierung, die Ablage in X-Y-Richtung (also Ablenkung des Teilchenstrahls in lateraler Richtung) der Durchmesser und/oder die Formgebung des Teilchenstrahls und dergleichen in Betracht. Der vorzugsweise bewegte Teilchenstrahl kann beispielsweise in Form eines Spot-Scanverfahrens (Teilchenstrahl wird zwischen zwei Rasterpositionen abgeschaltet; auf den einzelnen Rasterpositionen verweilt der Strahl eine bestimmte Zeit), eines Raster-Scanverfahrens (der Strahl verweilt jeweils an einem Rasterpunkt für eine unterschiedliche Zeit; ein Abschalten des Strahls zwischen zwei Rasterpositionen erfolgt dagegen nicht) oder eines kontinuierlichen Scanverfahrens (Teilchenstrahl wird kontinuierlich mit wechselnder Geschwindigkeit bewegt) bewegt werden. Der Dosiseintrag an einem Bestrahlungspunkt richtet sich insbesondere nach der Zeitdauer, in der Teilchen in den betreffenden Punkt eingetragen werden und/oder der Teilchenlumineszenz zu diesem Zeitpunkt. Grundsätzlich ist es jedoch auch möglich, dass ein unbewegter Teilchenstrahl verwendet wird, der entsprechend aufgeweitet ist und/oder durch geeignete Maßnahmen (beispielsweise unter Verwendung einer Paraffinplatte mit unterschiedlicher Dicke) einen hinsichtlich des Orts und der Energie aufgelösten, breiten Teilchenstrahl erzeugt. Während der Bestrahlungspunkt also vorzugsweise in Parametern vorliegt, die der Ansteuerung des Beschleunigers entsprechen, handelt es sich bei dem Zielpunktvolumen in der Regel um ein geometrisch vorliegendes Volumen (insbesondere im kartesischen Koordinatenraum). Die Umrechnung von Bestrahlungspunkten auf Zielpunktvolumen ist dabei komplex und insbesondere nicht linear. Denn im "geometrischen Raum", der beispielsweise durch bildgebende Verfahren (beispielsweise CT-Verfahren oder NMR-Verfahren; CT = Computertomographie; NMR = Nuclear Magnetic Resonance) ermittelt werden kann, hängt die effektive Reichweite eines Teilchenstrahls in einem Material (und damit unter anderem auch die Lage des Bragg-Peaks bei einem "angepeilten" Bestrahlungspunkt) in aller Regel von zusätzlichen Faktoren ab, insbesondere von der Dichte und/oder der Materialzusammensetzung des durchstrahlten Materials. Beispielsweise kann beim Durchstrahlen eines Lungenflügels ein luftgefüllter Hohlraum durchstrahlt werden (extrem geringe Dichte) und auf dem Weg gegebenenfalls auch eine Rippe durchstrahlt werden (hohe Dichte). Alle auf dem Weg des Teilchenstrahls liegenden Materialien haben einen starken Einfluss auf die effektive Lage des Bragg-Peaks, und damit auf die Verteilung der effektiv deponierten Dosis in den unterschiedlichen Zielpunktvolumen, und damit im geometrischen Raum. Die Art und die Dichteverteilung des Gewebes können sich im Rahmen einer Bewegung des bewegten Zielvolumenbereichs stark ändern. Beispielsweise kann durch die Atmung eines Patienten ein Stück Rippe aus der Teilchenlaufbahn des Teilchenstrahls heraus wandern und sich gleichzeitig das luftgefüllte Volumen in der Lunge (und damit die Wegstrecke, die durch Luft hindurch gelegt wird) stark verlängern bzw. verkürzen. Dementsprechend große Einflüsse ergeben sich hinsichtlich der Lage des Bragg-Peaks und/oder des Dosiseintrags in den Zielpunktvolumen. Vorteilhaft ist es, wenn zur Erstellung des Bestrahlungsplans eine größere Menge an Zielpunktvolumen verwendet wird, insbesondere eine Mannigfaltigkeit an Zielpunktvolumen, die einen größeren Bereich eines zu bestrahlenden Objekts (insbesondere zu bestrahlende Bereiche und/oder nicht zu bestrahlende Bereiche) umfasst. Bei den zu bestrahlenden Bereichen kann es sich insbesondere um das so genannte ITV (für "internal target volume") handeln. Bei dem ITV handelt es sich in der Regel um die Gebiete, die ein zu bestrahlendender Volumenbereich (meist als so genanntes CTV für "clinical target volume" bezeichnet) einnehmen kann, wenn man sämtliche (realistischerweise zu erwartenden) Bewegungszustände berücksichtigt. Es handelt sich also in der Regel um eine Art "geometrisches einhüllendes Volumen". Vorzugsweise wird die Größe derart gewählt, dass das durch den Teilchenstrahl erreichbare Volumen (plus einem Sicherheitszuschlag) in finite Zielpunktvolumenelemente (sogenannte Voxel) aufgelöst wird. Erreichbar ist insbesondere der Bereich des Objekts, der durch Ablenkungsmaßnahmen des Teilchenstrahls und/oder durch Energievariation des Teilchenstrahls erreicht werden kann (bzw. im Rahmen eines vorgesehenen Bestrahlungsvorhabens erreicht werden soll). Die Abbildungsfunktion kann auf im Wesentlichen beliebige Art und Weise vorliegen bzw. realisiert werden. Insbesondere ist es möglich, dass eine analytische Formel bzw. analytische Näherungsformel vorgesehen wird. Auch die Verwendung von Wertetabellen (bei denen die erwünschten Werte "nachgeschlagen" werden), Interpolationsverfahren und Mischungen aus den vorab genannten Verfahren sind denkbar. Insbesondere kann mithilfe der Abbildungsfunktion ein direktes "Mapping" ohne Umwege realisiert werden, wie beispielsweise ohne eine zunächst erfolgende Umrechnung in einen homogenen äquivalenten Phantomkörper, der im Folgenden für weitere Berechnungen verwendet wird. Dies schließt jedoch nicht aus, dass die Abbildungsfunktion "mehrteilig" vorliegt, wie beispielsweise unter Verwendung einer Transformationsfunktion (beispielsweise einer Transformationsmatrix) und einer in der Teilchentherapie oftmals verwendeten, so genannten HU-LUT (für Hounsfield-Unit look up table), mit der die Umrechnung von geometrischen Volumenbereichen in radiographische Volumenbereiche (bei denen unterschiedliche Materiedichten berücksichtigt werden; oftmals eine Umrechnung in so genannte "wasseräquivalente Körper"; in diesem Zusammenhang wird oftmals auch der Begriff "water-equivalent path length" (WEPL) verwendet) realisiert wird. Die Kombinationsfunktion einer derartigen Transformationsfunktion und einer HU-LUT kann dann die Abbildungsfunktion im Sinne dieser Anmeldung darstellen. Auch ist es möglich, dass für jeden einzelnen der unterschiedlichen Bewegungszustände jeweils ein eigenes ITV bestimmt wird. Zur gemeinsamen Optimierung aller derart erzeugten ITVs kann dann eine zusätzliche Transformationsfunktion (die nicht notwendigerweise mit der vorab beschriebenen übereinstimmen muss) verwendet werden, die die von der Einstrahlrichtung abhängigen Wasser-äquivalenten Reichweiten des betreffenden Zielvolumens in ein für alle Bestrahlungs-Felder in gleicher Weise gültiges, von der Einstrahlrichtung unabhängiges Zielvolumen überführt.

Insbesondere ist es von Vorteil, wenn das Verfahren derart durchgeführt wird, dass die Abbildungsfunktion zumindest die Reichweitenänderung des vorzugsweise bewegten Teilchenstrahls berücksichtigt, insbesondere die Reichweitenänderung des vorzugsweise bewegten Teilchenstrahls, der durch Dichteänderungen, insbesondere radiographische Dichteänderungen, in Teilchenstrahlrichtung bewirkt wird. Unter radiographischen Dichteänderungen kann insbesondere eine Dichteänderung in wasseräquivalenten Einheiten (WEPL - "water equivalent path length") verstanden werden. Insbesondere durch diese Weiterbildung des vorgeschlagenen Verfahrens ist es möglich, dass bei der Bestrahlungsplanung unterschiedlichste Arten von Bestrahlungsfeldern berücksichtigt und gleichzeitig optimiert werden können, ohne dass die Gefahr von unterdosierten Volumenbereichen übermäßig ansteigt. Insbesondere ist es möglich, dass ein Bestrahlungsplan erstellt werden kann, obwohl sowohl unterschiedliche Teilchenstrahl-Einstrahlungsrichtungen, als auch bewegte Zielvolumenbereiche vorliegen.

Besonders bevorzugt ist es, wenn die Abbildungsfunktion zusätzlich von einer Einstrahlrichtung des vorzugsweise bewegten Teilchenstrahls abhängt. Wie bereits erläutert, hängt die radiographische Dichte, und damit die schlussendliche Lage des Bragg-Peaks des Teilchenstrahls, bei bestimmten Objekten (insbesondere im medizinischen Bereich mit stark unterschiedlich dichten Materialbereichen) zum Teil stark von dem Weg ab, den der Teilchenstrahl durch das zu bestrahlende Objekt nimmt. Dementsprechend hängt die Lage des Bragg-Peaks insbesondere im medizinischen Bereich in der Regel stark von der Einstrahlrichtung des Teilchenstrahls ab. Wenn diese Abhängigkeit (zumindest teilweise) von der Abbildungsfunktion berücksichtigt wird, kann sich die Qualität des resultierenden Bestrahlungsplans zum Teil deutlich verbessern.

Vorteilhaft ist es weiterhin, wenn bei dem vorgeschlagenen Verfahren eine radiologische Wirksamkeit auf das mit der Strahlung zu beaufschlagende Material berücksichtigt wird. Insbesondere in der Medizin kann hier beispielsweise die so genannte RBE (für Relative Biological Effectivity) mit berücksichtigt werden.

Weiterhin wird vorgeschlagen, dass bei dem vorgeschlagenen Verfahren der Dosiseintrag in zumindest ein Zielpunktvolumen zumindest zeitweise und/oder zumindest teilweise für unterschiedliche Bewegungszustände und/oder für unterschiedliche Einstrahlrichtungen voneinander unterscheidbar ermittelt wird. Mit anderen Worten kann für jeden Bewegungszustand und/oder für jede unterschiedliche Einstrahlrichtung (wird beides berücksichtigt, kann insbesondere eine entsprechend große Matrix, die beide Effekte berücksichtigt, genutzt werden) die jeweils für diesen Zustand bzw. für diese Einstrahlrichtung resultierende Dosisverteilung im geometrischen Raum bestimmt werden. Es findet also vorzugsweise (zunächst) keine Mittelung aus unterschiedlichen Bewegungszuständen und/oder unterschiedlichen Einstrahlrichtungen oder dergleichen statt. Auch durch diese Weiterbildung kann ein in der Regel besonders guter Bestrahlungsplan realisiert werden.

Vorteilhaft ist es weiterhin, wenn bei dem Verfahren die Zielpunktvolumen zumindest teilweise als zu bestrahlende Zielpunktvolumen oder als nicht zu bestrahlende Zielpunktvolumen klassifiziert werden. Insbesondere kann für zu bestrahlende Zielpunktvolumen eine minimale Strahlungsdosis, die zu überschreiten ist und/oder für nicht zu bestrahlende Zielpunktvolumen eine maximale Strahlungsdosis, die nicht überschritten werden darf, definiert werden. Selbstverständlich ist es möglich, dass sowohl für zu bestrahlende, als auch für nicht zu bestrahlende Zielpunktvolumen in unterschiedlichen Bereichen unterschiedliche Grenzwerte definiert werden. Auf diese Weise ist es beispielsweise möglich besonders empfindliches Gewebe im Rahmen der Rechnung besonders zu berücksichtigen und/oder im Rahmen der Rechnung in besonderem Maße sicherzustellen, dass zu zerstörendes Gewebe (Material) tatsächlich zerstört wird.

Das Verfahren wird dahingehend durchgeführt, dass zur Berechnung des Bestrahlungsplans für eine Mehrzahl, vorzugsweise für alle Zielpunktvolumen, in unterschiedlichen Bewegungszuständen ein im Wesentlichen gleichartiges Bestrahlungsmuster verwendet wird. Mit anderen Worten kann für jeden Bewegungszustand die jeweils resultierende Dosisverteilung ermittelt werden, und die jeweils entstehenden Dosisverteilungen können durch geeignete mathematische Verfahren hinsichtlich ihrer Verteilung optimiert werden. Dieser Vorschlag basiert auf der Überlegung, dass sich bei einem nichtbewegungskompensierten Bestrahlungsverfahren der zu bestrahlende Zielvolumenbereich in einer beliebigen Bewegungsphase befinden kann. Dies ist dementsprechend bei der Berechnung zu berücksichtigen. Auch hierdurch können sich besonders vorteilhafte und genaue Bestrahlungspläne ergeben.

Weiterhin wird vorgeschlagen, dass bei dem Verfahren bei der Erstellung der Bestrahlungsplanung für die Mehrzahl von Bewegungszuständen eine Mehrzahl von Bewegungszuständen, die einen typischen Bewegungsablauf beschreiben, verwendet wird. Hierbei ist vorzugsweise ein guter Kompromiss zwischen einerseits besonders feiner Auflösung (und damit besseren Ergebnissen) und andererseits einer überschaubaren Rechenzeit zu finden. In der Praxis ist oftmals auch ausschlaggebend, wie viele Bewegungszustände zur Verfügung stehen bzw. zur Verfügung gestellt werden können (beispielsweise von medizinischem Fachpersonal, welches eine CT-Aufnahme angefertigt hat). Die Bewegungszustände und/oder der typische Bewegungsablauf können insbesondere durch bildgebende Verfahren vor der eigentlichen Bestrahlung gewonnen werden. Insbesondere bieten sich hierbei beispielsweise CT-Verfahren und/oder NMR-Verfahren an, welche insbesondere zeitaufgelöst arbeiten.

Möglich ist es jedoch auch, dass für die Erstellung des Bestrahlungsplans eine Unterauswahl von Bewegungszuständen verwendet wird. Hierdurch kann es ermöglicht werden die Rechenzeit zum Teil deutlich zu verringern, obgleich die Qualität des Bestrahlungsplans nicht deutlich verschlechtert wird. Eine besonders geeignete Unterauswahl von Bewegungszuständen sind beispielsweise Bewegungszustände, die besonders häufig und/oder über besonders lange Zeiträume hinweg eingenommen werden und/oder Bewegungszustände, die Extrempositionen darstellen (beispielsweise bei einem Atemzyklus ein voll eingeatmeter und ein voll ausgeatmeter Zustand).

Besonders vorteilhaft ist es, wenn zumindest Teilen der Zielpunktvolumen eine Solldosis und/oder eine Toleranzdosis zugeordnet ist. Derartige Werte können als besonders geeignete Input-Parameter für eine automatisierte Optimierung des Bestrahlungsplans genutzt werden. Eine Toleranzdosis kann dabei auch in Form eines unteren Grenzwerts und/oder eines oberen Grenzwerts vorliegen.

Als in der Praxis besonders leicht zu handhaben hat es sich erwiesen, wenn bei dem Verfahren als Abbildungsfunktion zumindest zeitweise und/oder zumindest bereichsweise eine Datenmatrix verwendet wird. Hierdurch kann (insbesondere im Verhältnis zu einer analytisch vorliegenden Funktion) in der Regel Rechenzeit eingespart werden. Gerade Rechenzeit ist bei heutigen Computern eher ein Problem, als Speicher. Dabei ist darauf hinzuweisen, dass selbst für komplexe Systeme mit mehreren zehntausend Zielpunktvolumen lediglich Speicherbedarf im Bereich von Gigabyte erforderlich ist, was mit heutzutage kommerziell erhältlichen Computern vergleichsweise einfach und kostengünstig realisiert werden kann.

Weiterhin ist es von Vorteil, wenn bei dem Verfahren eine Optimierung in Bezug auf die Solldosis-Verteilung erfolgt. Insbesondere kann die Optimierung automatisiert erfolgen, speziell durch an sich bekannte Optimierungsverfahren in multidimensionalen Systemen. Insbesondere ist es möglich, dass unter Verwendung der Abbildungsfunktion eine direkte Optimierung eines einzelnen, gemeinsamen ITV realisiert werden kann. Zusätzlich oder alternativist es aber auch möglich, dass für jede einzelne Bewegungsphase ein jeweiliges, vorzugsweise optimiertes ITV bestimmt wird, welches das Zielvolumen in diesem Bewegungszustand abdeckt. Zur gemeinsamen Optimierung aller derart gewählten ITV kann eine Transformationsvorschrift verwendet werden, die das jeweilige ITV in ein von der Einstrahlrichtung unabhängiges, gemeinsames Zielvolumen überführt. Letzteres kann dann (nochmals) optimiert werden.

Eine weitere vorteilhafte Weiterbildung des Verfahrens kann sich ergeben, wenn bei der Berechnung des Bestrahlungsplans zumindest zwei Bewegungszustände und/oder zumindest zwei Richtungen des Strahlungseintrags berücksichtigt werden. Vorteilhaft ist es jedoch, wenn eine größere Anzahl an Bewegungszuständen und/oder Richtungen des Strahlungseintrags berücksichtigt werden, da dadurch in der Regel die Qualität des Bestrahlungsplans steigt.

Weiterhin wird eine Vorrichtung zur Erstellung einer Bestrahlungsplanung zur Bestrahlung eines bewegten Zielvolumenbereichs mit einem vorzugsweise bewegten Teilchenstrahl vorgeschlagen, wobei die Vorrichtung derart ausgebildet und eingerichtet ist, dass diese ein Verfahren mit den vorab beschriebenen Eigenschaften durchführt. Eine derartige Vorrichtung kann dann die bereits vorab beschriebenen Vorteile und Eigenschaften zumindest in analoger Weise aufweisen. Weiterhin kann die Vorrichtung im Sinne der vorherigen Beschreibung zumindest in Analogie weitergebildet werden.

Besonders vorteilhaft ist es in der Regel, wenn die Vorrichtung derart ausgebildet ist, dass diese zumindest eine programmtechnisch gesteuerte Recheneinheit aufweist. Insbesondere ist an Computer (beispielsweise Personal Computer und/oder Workstations und/oder Großcomputer) zu denken. Derartige Vorrichtungen erweisen sich aufgrund der leichten Programmierbarkeit und der leichten kommerziellen Erhältlichkeit als für die Durchführung des Verfahrens besonders geeignet.

Im Folgenden wird die Erfindung anhand vorteilhafter Ausführungsbeispiele und unter Bezugnahme auf die beigefügte Zeichnung näher erläutert. Es zeigen:
- Fig. 1:: ein denkbares Beispiel für eine Abbildungsfunktion zwischen Bestrahlungspunkt und Zielpunktvolumen für eine Mehrzahl von Bewegungsphasen;
- Fig. 2:: Effekte auf den Gesamtdosiseintrag bei Verwendung unter-schiedlicher Einstrahlrichtungen unter Anwendung unter-schiedlicher Bestrahlungsplan-Optimierungsverfahren;
- Fig. 3:: der Einfluss von unterschiedlicher Dichte von Gewebe beim Vorhandensein von Bewegungen auf den Ort der deponierten Dosis;
- Fig. 4:: ein schematisches Ablaufdiagramm für ein Verfahren zur Erstellung eines Bestrahlungsplans;
- Fig.5:: ein dose-volume-histogram (DVH) von verschiedenartigen Bestrahlungsverfahren an einem Prototypen.

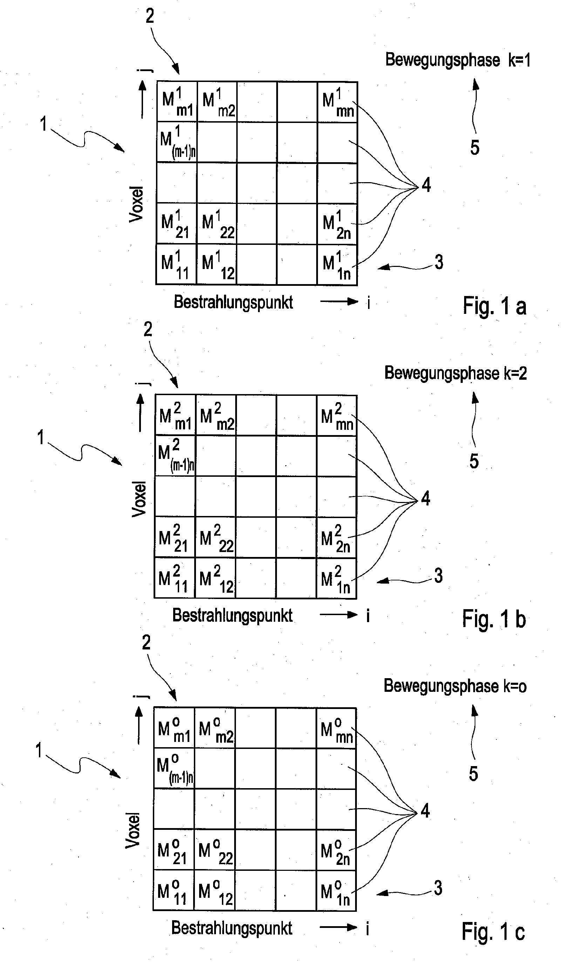

In Fig. 1 ist als Beispiel für eine mögliche Abbildungsfunktion, mit der der durch die Beaufschlagung eines Bestrahlungspunkts Bᵢ bewirkte effektive Dosiseintrag in unterschiedliche Volumenbereiche Vⱼ realisiert werden kann, gemäß einem denkbaren Ausführungsbeispiel, dargestellt. Die Abbildungs- funktion ist vorliegend als (mehrdimensionale) Matrix 1 ausgebildet. Die Dimension der Matrix 1 bestimmt sich dabei aus der Anzahl der zu berücksichtigenden Bewegungsphasen (in Fig. 1 sind diese unterschiedlichen Bewegungsphasen-"Ebenen" untereinander dargestellt).

Für die Erstellung der Abbildungsmatrix 1 wird ein zu bestrahlender Körper in eine größere Anzahl an einzelnen finiten Volumenelementen, so genannten Voxeln Vⱼ aufgeteilt. Der in derartige Voxel Vⱼ aufzuteilende Volumenbereich des Zielobjekts ist dabei größer gewählt, als das eigentlich mit einer Strahlung zu beaufschlagende Zielvolumen (beispielsweise ein zu therapierender Tumor bzw. ein entsprechender Zielvolumenbereich eines Bestrahlungsphantoms), einschließlich der durch eine (innere) Bewegung des eigentlichen Zielvolumenbereichs bewirkten Vergrößerung des zu bestrahlenden Gebiets und/oder unter Berücksichtigung eines Sicherheitssaums. Denn bei der Erstellung eines Bestrahlungsplans erweist es sich als sinnvoll, wenn auch die üblichen Bewegungen des Zielobjekts bei der Erstellung des Bestrahlungsplans berücksichtigt werden. Dadurch kann eine Optimierung des Bestrahlungsplans sowohl in Bezug auf einen ausreichenden Dosiseintrag in mit einer Dosis zu beaufschlagenden Volumenbereich, als auch in Bezug auf einen möglichst niedrigen Dosiseintrag in an sich nicht mit einer Dosis zu . beaufschlagenden Volumenbereich (was jedoch in aller Regel nicht vollständig vermieden werden kann) ermöglicht werden. Besonders relevant ist insbesondere eine Vermeidung eines Dosiseintrags in bestimmte Volumenbereiche, wenn es sich um besonders empfindliches und/oder aus sonstigen Gründen besonders zu schützendes Gewebe (oftmals als so genanntes OAR = Organ at Risk bezeichnet) handelt. Umgekehrt ist es im Übrigen nicht notwendigerweise erforderlich, dass das gesamte zu bestrahlenden Objekt bei der Aufteilung in einzelne Voxel Vⱼ berücksichtigt werden muss. Wenn ein im Vergleich zum gesamten Objekt vergleichsweise kleines zu bestrah-lendes Zielgebiet vorliegt, so steht üblicherweise das Ausmaß der Verbesserung des Bestrahlungsplans in keinem Verhältnis zum erhöhten Rechenaufwand, wenn weit entfernte Gebiete bei der Erstellung des Bestrahlungsplans mit berücksichtigt werden. Ein Beispiel hierfür ist beispielsweise die Therapie eines in der Lunge gelegenen Tumors und die Berücksichtigung der Beine eines Patienten (was in aller Regel nicht sinnvoll ist).

Je nach Größe des gewählten Volumens und der Unterteilung desselben in einzelne Voxel Vⱼ (Genauigkeit) ergibt sich eine entsprechende Anzahl n an Voxeln Vⱼ. Diese Anzahl n an einzelnen Voxeln Vⱼ hat einen Einfluss auf die Größe der resultierenden Abbildungsmatrix 1.

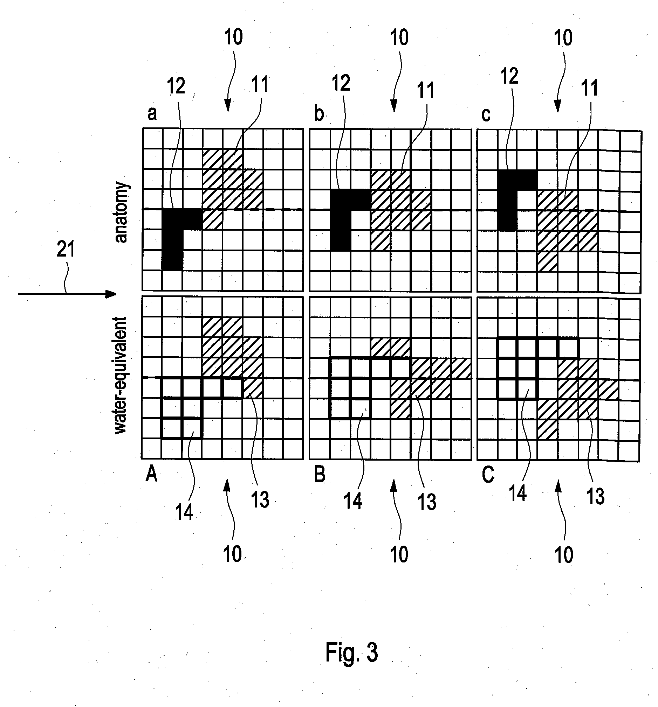

Weiterhin wird eine Menge an Bestrahlungspunkten Bᵢ vorgegeben, die von dem Teilchenstrahl im Rahmen der Bestrahlung angefahren werden. Hierbei ist es ebenfalls nicht zwingend erforderlich, dass sämtliche physikalisch mit der jeweiligen Anlage erzielbaren Bereiche (insbesondere x- und y-Ablenkungs- sowie Energievariationen, was der z-Ablenkung entspricht) berücksichtigt werden müssen. Stattdessen werden üblicherweise Parametersätze verwendet, welche in einem sinnvollen Verhältnis zum zu therapierenden Volumen stehen. Die Bestrahlungspunkte Bᵢ entsprechen dabei in gewissem Maße den Parametern des Teilchenstrahls, also insbesondere Ablenkung des Strahls und Energie der Teilchen des Teilchenstrahls. Die Anzahl m an Bestrahlungspunkten Bᵢ hat dabei ebenfalls einen Einfluss auf die Größe der Abbildungsmatrix 1. Wird nunmehr ein einzelner Bestrahlungspunkt Bᵢ von einem Teilchenstrahl "angefahren", so bewirkt dies nicht nur einen Strahlungseintrag in ein einzelnes Voxel Vⱼ (bzw. in eine sehr kleine Anzahl unmittelbar nebeneinanderliegender Voxel Vⱼ), sondern es kommt auch zu einem Strahlungseintrag in entfernter liegende Voxel Vⱼ. Insbesondere kommt es zu einem Strahlungseintrag in Voxeln Vⱼ, welche in Bereichen liegen, die der Teilchenstrahl auf dem Weg zu seinem Ziel durchläuft (proximal liegende Bereiche). Darüber hinaus hat die Art des durchstrahlten Gewebes einen Einfluss auf die effektive Länge des Teilchenstrahls im Objekt. Handelt es sich beispielsweise um ein dichtes Gewebe (insbesondere wasserreiches Gewebe), so verkürzt sich die Weglänge, die der Teilchenstrahl zurücklegt, bis er "stecken bleibt". Liegen dagegen Bereiche mit geringer Dichte vor (beispielsweise luftgefüllte Hohlräume, zum Beispiel eine Lunge in eingeatmetem Zustand), so liegt der Teilchenstrahl bei gleicher Energie einen deutlich größeren Weg im Objekt zurück. Dieser Zusammenhang wird im Folgenden noch unter Bezugnahme auf Fig. 3 näher erläutert.

Beide Effekte, also sowohl die Eindringtiefe des Teilchenstrahls (und damit der Zusammenhang zwischen dem geometrischen Volumen - wie es beispielsweise durch bildgebende Verfahren ermittelt werden kann - und dem radiologischen Ort unter Berücksichtigung unterschiedlicher Materiedichte), als auch der Dosiseintrag in entfernter liegende Volumenbereiche (Voxel Vⱼ) wird durch die Größe der jeweiligen Matrixelemente Mᵢⱼ^{k} beschrieben. Ein einzelner Koeffizient Mᵢⱼ^{k} beschreibt also den Dosiseintrag in das j-te Voxel, wenn der i-te Bestrahlungspunkt angefahren wird. Die einzelnen Koeffizienten sind dabei hinsichtlich der Teilchenanzahl normiert. Bei der Erstellung eines Bestrahlungsplans wird daher der vorgesehene Bestrahlungsplan rechnerisch "getestet" und die mithilfe der Abbildungsmatrix 1 gewonnene effektive Verteilung im geometrischen Volumen (in den Voxeln Vⱼ) ermittelt. Je nach Teilchenfluenz (also applizierte Teilchenanzahl pro Bestrahlungspunkt) müssen die einzelnen Koeffizienten Mᵢⱼ^{k} entsprechend der Teilchenfluenz multipliziert werden.

Die Verteilung der Koeffizienten variiert jedoch beim Vorhandensein einer (inneren) Bewegung im Zielbereich des zu bestrahlenden Objekts. Denn durch eine innere Bewegung kommt es nicht nur zu einer geometrischen Verschiebung des entsprechenden Zielbereichs, sondern darüber hinaus noch zu Dichtänderungen im umliegenden Material (Gewebe). Dies hat entsprechende Effekte auf die Eindringtiefe des Teilchenstrahls.

Um diese Effekte zu berücksichtigen wird daher nicht nur eine einzelne Abbildungsmatrix 1 für eine bestimmte Bewegungsphase k (in Fig. 1a eine Bewegungsphase k = 1) verwendet, sondern es werden vielmehr mehrere einzelne Abbildungsmatrizen 1 verwendet, die jeweils unterschiedliche Bewegungsphasen k beschreiben. Die Bewegungsphasen k (und die jeweilige Zielvolumenbereichsänderung) können beispielsweise durch bildgebende Verfahren, wie CT-Verfahren und/oder NMR-Verfahren vorab gewonnen werden. Die Anzahl der Bewegungsphasen k = 1...o ist dabei sinnvoll zu wählen. Insbesondere sollte die Ortsänderung, die durch die Bewegung zwischen zwei einzelnen Bewegungsphasen k bewirkt wird, in einem sinnvollen Verhältnis (typischerweise im Bereich von 1) zur Auflösung (Größe) der einzelnen Voxel Vⱼ stehen.

Somit ergibt sich eine Vielzahl von Abbildungsmatrizen 1 für die jeweiligen unterschiedlichen Bewegungsphasen k = 1...o.

Lediglich der Vollständigkeit halber sollte darauf hingewiesen werden, dass es natürlich ebenso möglich ist, eine einzelne Abbildungsmatrix entsprechend größer auszuführen (also in der Regel o-mal so groß), so dass diese (trotz gleicher Dimension) die erforderlichen Koeffizienten aufnehmen kann.

Lediglich der Vollständigkeit halber sollte darauf hingewiesen werden, dass die Anzahl der Bewegungsphasen k reduziert werden kann, wenn das Verfahren in Kombination mit Gating-Verfahren genutzt wird. Bei Gating-Verfahren wird eine Dosis nur dann appliziert, wenn sich das Zielvolumen in bestimmten Bewegungsphasen k befindet. Für die Bestrahlungsplanung müssen dann selbstverständlich lediglich diese Bewegungsphasen berücksichtigt werden.

Auch die Einstrahlungsrichtung des Teilchenstrahls in das Zielvolumen kann einen Effekt auf die Verteilung der Koeffizienten der Abbildungsmatrix 1 haben. Dementsprechend ist die Abbildungsmatrix 1 gegebenenfalls geeignet zu erweitern.

Bei der Berechnung eines Bestrahlungsdurchgangs im Rahmen der Bestrahlungsplanung wird dabei nicht, wie dies bislang im Stand der Technik oftmals gemacht wird, ein einzelner Voxel-Vektor V^{k} zur Beschreibung der Zielvorgabe in jedem Voxel-Element Vⱼ mit j = 1...n verwendet. Stattdessen wird für jede Bewegungsphase ein einzelner Voxel-Vektor V^{k} verwendet. Die Zielvorgabe wird dabei jedoch nicht verändert, so dass die Anforderungen der Dosisbelegung von einer Bewegungsphase auf alle Bewegungsphasen übertragen werden. Die Länge des Vektors der Teilchenbelegungen (Bestrahlungspunkte Bᵢ) bleibt dabei unverändert. Die Voxel-Vektoren V^{k} werden über die Abbildungsmatrizen mit den Einträgen Mᵢⱼ^{k} mit dem Vektor B der Teilchenüberlegungen verknüpft. Dieses Vorgehen basiert auf der Überlegung, dass bei einer Bestrahlung ohne Tracking (gegebenenfalls auch ohne Gating) davon auszugehen ist, dass sich das eigentliche Zielvolumen in einer beliebigen Bewegungsphase befinden darf, während die Bestrahlung erfolgt. (Bei Verwendung von Gating-Verfahren ist sinngemäß eine Beschränkung auf bestimmte Bewegungsphasen gegeben).

Bei der anschließenden Optimierung des Bestrahlungsplans wird anschließend versucht, die Teilchenbelegungen Bᵢ derart zu wählen (zu optimieren), das diese hinsichtlich der Zielvorgaben (Soll-Dosis) aus den einzelnen Voxel-Vektor-Angaben V^{k} optimiert sind.

Die Zielvorgabe kann dabei insbesondere eine bestimmte Solldosis pro Voxel (einschließlich eines tolerierbaren Schwankungswerts) darstellen. Insbesondere ist es auch möglich, dass die Solldosis und/oder die tolerierbare Schwankungsbreite mit einem gewissen Minimalwert nach unten versehen sind und/oder mit einem gewissen Maximalwert nach oben (insbesondere für OAR) versehen sind.

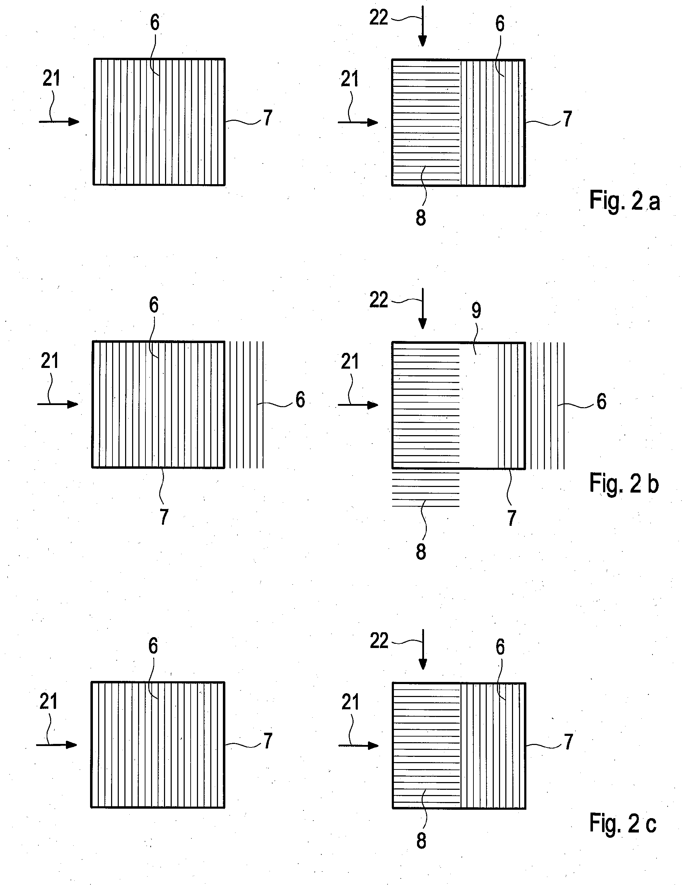

Der große Vorteil des beschriebenen Verfahrens besteht darin, dass insbesondere eine Mehrfeld-Optimierung möglich wird, ohne dass es zu unerwünschten Über- bzw. Unterdosierungen kommen muss. Dies ist unter Bezugnahme auf Fig. 2 näher erläutert. Die jeweiligen Figs. 2a - 2c zeigen jeweils links eine Einfeld-Bestrahlung und rechts eine Zweifeld-Bestrahlung mit einem Strahlungseintrag 21, 22 aus unterschiedlichen Richtungen.

In Fig. 2a ist eine Dosisverteilung 6 (senkrechte Striche) durch einen Strahlungseintrag 21 von links (durch Pfeil angedeutet) durch senkrechte Striche angedeutet. Das Zielgebiet 7 befindet sich dabei in einer Referenzphase, auf die der Bestrahlungsplan optimiert ist. In dieser Referenzphase ist es auch möglich, dass ein Dosiseintrag aus zwei unterschiedlichen Richtungen 21, 22 (durch Pfeile angedeutet) erfolgt. Die Bestrahlungsplanung ist dabei so gewählt, dass sich der durch den in der Zeichnung links liegenden Teilchenstrahl 21 bewirkte erste Dosiseintrag 6 (senkrechte Striche) und der durch den von oben kommenden Teilchenstrahl 22 bewirkte zweite Dosiseintrag 8 (waagerechte Striche) zu einem nahtlosen Dosiseintrag ergänzen.

Kommt es nun jedoch zu einer Verringerung der Dichte im Zielgebiet 7, so nimmt die Reichweite eines Teilchenstrahls 21, 22 zu. Dementsprechend "verlängert sich" das Gebiet des ersten Dosiseintrags 6 korrespondierend zur Dichteabnahme. Der "linke Rand" des ersten Dosiseintragsgebiets 6 bleibt unverändert, da vorliegend davon ausgegangen wird, dass außerhalb des Zielgebiets 7 keine Dichteänderung erfolgt. Im Einfeld-Fall (in der Zeichnung links) kommt es damit zu einer unerwünschten Dosisbeaufschlagung in an sich nicht zu beaufschlagendem Material, welches sich rechts vom Zielgebiet 7 befindet. Bereits dieser Effekt ist unerwünscht. Der in der Regel bedeutendere Nachteil, dass es zu einer Unterdosierung in zu bestrahlendem Material kommen kann (was beispielsweise bei einer Krebsbehandlung den Therapieerfolg dadurch gefährden kann, dass Krebszellen nicht vernichtet werden) tritt jedoch nicht auf.

Werden jedoch zwei unterschiedliche Teilchen-Einstrahlrichtungen verwendet, so verlängern sich nicht nur die entsprechenden Dosiseintrags-Felder 6, 8. Da die "linke Kante" des ersten Dosiseintrags-Felds 6 nunmehr in der Mitte des Zielgebiets 7 liegt (also einem Bereich, in dem es zu einer Dichtevariation gekommen ist), verschiebt sich dementsprechend die "linke Kante" des Dosis-Eintragsfelds 6. Dementsprechend kommt es zu einem unterdosierten Bereich 9, der - wie bereits erwähnt - im Falle einer Krebstherapie den Therapieerfolg massiv gefährdet.

Führt man jedoch für den in Fig. 2b dargestellten Bewegungszustand eine eigene Optimierung gemäß dem bereits vorab erläuterten Verfahren durch, so können wieder homogene Strahlungseinträge, insbesondere solche ohne Unterdosierung, realisiert werden. Dies ist in Fig. 2c angedeutet. Gegebenenfalls kann es auch zu einer (etwas) stärkeren Dosis im Grenzgebiet zwischen dem ersten Dosiseintrags-Feld 6 und dem zweiten Dosiseintrags-Feld 8 kommen.

In Fig. 3 ist nochmals die Veränderung der Reichweite eines Teilchenstrahls aufgrund von Dichteveränderungen dargestellt.

In der ersten Zeile ist ein zu bestrahlendes Zielgebiet 10 in drei unterschiedlichen Bewegungsphasen a, b, c dargestellt. Im Zielgebiet 10 ist ein sich bewegendes, mit einer Dosis zu beaufschlagende Zielvolumen 11 eingezeichnet. Weiterhin ist, dem Zielvolumen 11 proximal vorgelagert, ein sich ebenfalls bewegender Bereich geringer Dichte 12 vorhanden.

Da der Bereich der geringen (radiologischen) Dichte 12 die Reichweite des Teilchenstrahls ändert ergibt sich "aus Sicht des Teilchenstrahls" ein unterschiedliches zu bestrahlendes Zielvolumen 13. In Fig. 3 ist aus Gründen der Einfachheit angenommen, dass ein Volumenelement im Bereich höherer Dichte 12 für den Teilchenstrahl gesehen eine im Verhältnis zum sonstigen Gewebe doppelt so große radiologische Dichte aufweist. Dementsprechend entspricht ein Volumenelement im Bereich höherer Dichte 12 im geometrischen Raum zwei Volumenelementen 14 im "radiologischen Koordinatenbild".

Wie man sieht, verändert sich das Zielvolumen 13 im radiologischen Bild stark mit den unterschiedlichen Bewegungsphasen A, B, C (untere Zeile in Fig. 3).

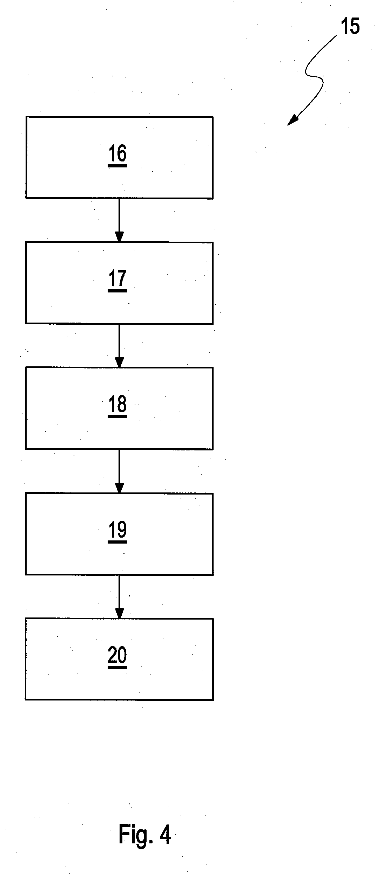

In Fig. 4 ist das vorgeschlagene Verfahren kurz in Form eines Ablaufdiagramms 15 skizziert. Zunächst wird in einem ersten Schritt 16 die geometrische Lage des zu bestrahlenden Zielbereichs 11 (gegebenenfalls auch die geometrische Lage von nicht zu bestrahlenden Gebieten wie OARs) in den unterschiedlichen Bewegungsphasen bestimmt. Hierzu eignen sich beispielsweise im medizinischen Bereich CT- und NMR-Verfahren.

Hat man diese Information gewonnen, so wird das im Rahmen der Bestrahlung zu bestrahlende Volumen in den unterschiedlichen Bewegungsphasen bestimmt (Schritt 17). Im medizinischen Bereich spricht man hierbei vom so genannten CTV (= Clinical Target Volume). Dieses ist in der Regel um so genannte Sicherheitssäume vergrößert, so dass die zu realisierenden minimalen Dosen im zu bestrahlenden Bereich sicher erreicht werden (auch wenn dies gegebenenfalls eine gewisse Schädigung von gesundem Gewebe zur Folge haben kann).

Anschließend wird im Schritt 18 die Abbildungsmatrix (beispielsweise Abbildungsmatrix 1 wie in Fig. 1) erstellt.

Unter Verwendung dieser Abbildungsmatrix werden nun jeweils einzelne "Test-Bestrahlungspläne" durchgerechnet und durch Optimierungsalgorithmen optimiert (Schritt 19). Auf diese Weise erhält man schlussendlich einen optimierten Bestrahlungsplan, der im Schritt 20 ausgegeben wird.

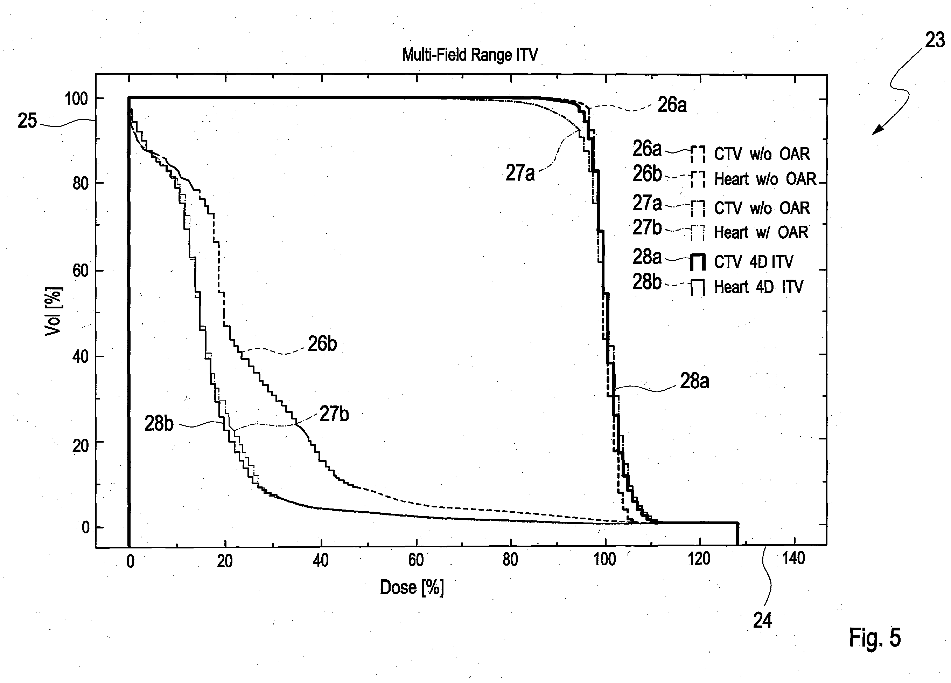

In Fig. 5 ist ein DVH (dose volume histogram) 23 gezeigt, bei dem der Dosiseintrag in einen Prototypen für unterschiedliche Bestrahlungsverfahren dargestellt ist. Simuliert ist dabei ein Tumor, der sich an der Lunge befindet. Bei einem DVH 23 wird längs der Abszisse 24 der Dosiseintrag in Prozent dargestellt, während längs der Ordinate 25 der Anteil des Gewebes dargestellt wird, der mit der entsprechenden Dosis beaufschlagt wird. Ein idealer Dosiseintrag für zu bestrahlende Gewebebereiche wäre eine Rechteck-Linie längs der 100 Volumenprozent-100 Dosisprozent-Linie. Dementsprechend wäre für nicht zu bestrahlende Gewebebereiche ein idealer Strahlungseintrag eine Rechteck-Linie längs der 0 Volumenprozent-0 Dosisprozent-Linie. Dargestellt sind drei Bestrahlungsverfahren (26, 27, 28) wobei jeweils rechts oben in Fig. 5 zu bestrahlende Gewebebereiche (26a, 27a, 28a) und links unten in Fig. 5 nicht zu bestrahlende Gewebebereiche (26b, 27b, 28b) eingezeichnet sind.

Linie 26 stellt ein Bestrahlungsergebnis dar, das mit einem Mehrfeld-ITV-Bestrahlungsplan erzielt wurde (ITV = internal target volume). Wie man an der Linie 26a für zu bestrahlende Gewebebereiche sieht, ist eine gute Dosisbelegung für zu bestrahlende Gewebebereiche gegeben. Dies wird jedoch mit einem hohen Dosiseintrag in nicht zu bestrahlende Gewebebereiche (Linie 26b) erkauft. Bei empfindlichen Organen (insbesondere OAR = organ at risk) ist dies nicht hinnehmbar.

Eine Verbesserung ergibt sich, wenn man einen Mehrfeld-ITV-Bestrahlungsplan, der OARs (vorliegend das Herz) berücksichtigt, verwendet. Das Ergebnis sieht man an den Linien 27. Deutlich ist zu erkennen, dass der Dosiseintrag in nicht zu bestrahlende Gewebebereiche (Linie 27b) deutlich reduziert ist. Gleichzeitig sieht man jedoch auch, dass zum Teil eine schlechte Dosisbelegung in eigentlich zu bestrahlende Gewebebereichen (Unterdosierung) gegeben ist, was ebenfalls problematisch ist (Linie 27a).

Deutlich bessere Ergebnisse sowohl für zu bestrahlende Gewebebereiche, als auch für nicht zu bestrahlende Gewebebereiche erhält man bereits, wenn man eine Mehrfeld-ITV-Bestrahlungsplanung nutzt, bei der (nur!) zwei Bewegungsphasen (vorliegend voll eingeatmet und voll ausgeatmet) berücksichtigt werden. Dies ist an der durchgehenden schwarzen Linie 28 zu erkennen. Sowohl die Dosisbelegung in zu bestrahlende Gewebebereiche (Linie 28a) ist sehr gut, als auch die (geringe) Dosisbelegung in nicht zu bestrahlende Gewebebereiche (Linie 28b).

### Bezugszeichenliste:

1. Abbildungsmatrix
2. Voxel-Vektor
3. Bestrahlungspunkt-Vektor
4. Matrix-Koeffizient
5. Bewegungsphase
6. Erstes Dosis-Feld
7. Zielgebiet
8. Zweites Dosis-Feld
9. Unterdosierter Bereich
10.Zielgebiet
11.Zielvolumen
12. Bereich hoher Dichte (geometrische)
13. Zielvolumen (radiologisch)
14. hohe Dichte (radiologisch)
15.Ablaufdiagramm
16. Bestimmung ITV
17. Bestimmung CTV
18. Erstellung der Abbildungsmatrix
19. Optimierung des Bestrahlungsplans
20. Ausgabe des Bestrahlungsplans
21. Strahlungseintrag von links
22. Strahlungseintrag von oben
23. DVH (dose volume histogram)
24. Abszisse
25. Ordinate
26. Mehrfeld-ITV ohne OAR
27. Mehrfeld-ITV mit OAR
28. Mehrfeld-ITV mit OAR und zwei Bewegungsphasen

## Patentansprüche

1. Verfahren (15) zur Erstellung eines Bestrahlungsplans zur Bestrahlung eines zumindest bereichsweise bewegten Zielvolumenbereichs (11, 13) in einem Objekt (10) mit einem Teilchenstrahl, wobei der Bestrahlungsplan Bestrahlungspunkte (Bᵢ) umfasst, die vom Teilchenstrahl im Rahmen der Bestrahlung angefahren werden und die jeweils einen Satz an Ansteuerungsparametern für den Teilchenstrahl darstellen, das Verfahren umfassend die Schritte
- Vorgeben einer Mehrzahl von Bewegungszuständen (k) für den zu bestrahlenden Zielvolumenbereich;
- Bestimmen des zu bestrahlenden Volumens für jeden der Bewegungszustände (k);
- Festlegen einer Mehrzahl von Zielpunktvolumen (Vⱼ) im zumindest bereichsweise bewegten Zielvolumenbereich;
- Erstellen einer Abbildungsfunktion (1) zwischen den Bestrahlungspunkten (Bᵢ) und den Zielpunktvolumen (Vⱼ), wobei die Abbildungsfunktion (1) Werte enthält, die für jeden der Mehrzahl an Bewegungszuständen (k) den Dosiseintrag in zumindest ein Zielpunktvolumen (Vⱼ) beschreiben, wenn der i-te Bestrahlungspunkt (Bᵢ) angefahren wird; und
- Erstellen des Bestrahlungsplans unter Verwendung der Abbildungs-funktion (1),
**dadurch gekennzeichnet, dass** zur Berechnung des Bestrahlungsplans für eine Mehrzahl von Zielpunktvolumen (Vⱼ) in unterschiedlichen Bewegungszuständen (k) ein im Wesentlichen gleichartiges Bestrahlungsmuster verwendet wird.

2. Verfahren (15) nach Anspruch 1, **dadurch gekennzeichnet, dass** der Teilchenstrahl ein bewegter Teilchenstrahl ist.

3. Verfahren (15) nach Anspruch 1 oder 2, **dadurch gekennzeichnet, dass** die Abbildungsfunktion (1) zumindest die Reichweitenänderung des Teilchenstrahls berücksichtigt.

4. Verfahren (15) nach Anspruch 3, **dadurch gekennzeichnet, dass** die Reichweitenänderung des Teilchenstrahls, die durch Dichteänderungen bewirkt wird, berücksichtigt wird.

5. Verfahren (15) nach einem der vorangehenden Ansprüche, **dadurch gekennzeichnet, dass** die Abbildungsfunktion (1) zusätzlich von einer Einstrahlrichtung des vorzugsweise bewegten Teilchenstrahls abhängt.

6. Verfahren (15) nach einem der vorangehenden Ansprüche, **dadurch gekennzeichnet, dass** der Dosiseintrag in zumindest ein Zielpunktvolumen (Vⱼ) zumindest teilweise für unterschiedliche Bewegungszustände (k) und/oder für unterschiedliche Einstrahlrichtungen voneinander unterscheidbar ermittelt wird.

7. Verfahren (15) nach einem der vorangehenden Ansprüche, **dadurch gekennzeichnet, dass** die Zielpunktvolumen (Vⱼ) zumindest teilweise als zu bestrahlende Zielpunktvolumen (Vⱼ) oder als nicht zu bestrahlende Zielpunktvolumen (Vⱼ) klassifiziert sind.

8. Verfahren (15) nach einem der vorangehenden Ansprüche, **dadurch gekennzeichnet, dass** bei der Erstellung der Bestrahlungsplanung eine Mehrzahl von Bewegungszuständen (k), die einen typischen Bewegungsablauf beschreiben, verwendet wird.

9. Verfahren (15) nach einem der vorangehenden Ansprüche, insbesondere nach Anspruch 9, **dadurch gekennzeichnet, dass** für die Erstellung des Bestrahlungsplans eine Unterauswahl von Bewegungszuständen (k) verwendet wird.

10. Verfahren (15) nach einem der vorangehenden Ansprüche, **dadurch gekennzeichnet, dass** zumindest Teilen der Zielpunktvolumen (Vⱼ) eine Solldosis und/oder eine Toleranzdosis zugeordnet ist.

11. Verfahren (15) nach einem der vorangehenden Ansprüche, **dadurch gekennzeichnet, dass** als Abbildungsfunktion (1) oder zumindest bereichsweise eine Datenmatrix (1) verwendet wird.

12. Verfahren (15) nach einem der vorangehenden Ansprüche, **dadurch gekennzeichnet, dass** eine Optimierung in Bezug auf eine Soll-Dosisverteilung erfolgt.

13. Verfahren (15) nach einem der vorangehenden Ansprüche, **dadurch gekennzeichnet, dass** bei der Berechnung des Bestrahlungsplans zumindest zwei Bewegungszustände (k) oder zumindest zwei Richtungen des Strahlungseintrags berücksichtigt werden.

14. Vorrichtung zur Erstellung einer Bestrahlungsplanung zur Bestrahlung eines bewegten Zielvolumenbereichs (11, 13) mit einem vorzugsweise bewegten Teilchenstrahl, **dadurch gekennzeichnet, dass** diese derart ausgebildet und eingerichtet ist, dass diese ein Verfahren (15) nach einem der Ansprüche 1 bis 13 durchführt.

15. Vorrichtung nach Anspruch 14, **dadurch gekennzeichnet, dass** diese zumindest einen programmtechnisch gesteuerte Recheneinheit aufweist.

## Claims

1. Method (15) for creating an irradiation plan for irradiating a target volume area (11, 13), moving in at least some areas, inside an object (10) using a particle beam, the irradiation plan comprising irradiation points (Bᵢ) which are approached by the particle beam in the course of irradiation and which each represent a set of control parameters for the particle beam, the method comprising the steps
- Predefining a plurality of movement states (k) for the target volume area to be irradiated;
- Determining the volume to be irradiated for each of the movement states (k);
- Stipulating a plurality of target point volumes (Vⱼ) in the target volume area moving in at least some areas;
- Creating a mapping function (1) between the irradiation points (Bᵢ) and the target point volumes (Vⱼ), the mapping function (1) containing values which describe, for each of the plurality of movement states (k), the dose administered to at least one target point volume (Vⱼ) when the i-th irradiation point (Bᵢ) is approached; and
- Creating the irradiation plan using the mapping function (1),
**characterized in that** a substantially similar irradiation pattern is used to calculate the irradiation plan for a plurality of target point volumes (Vⱼ) in differing movement states (k).

2. Method (15) according to claim 1, **characterized in that** the particle beam is a moving particle beam.

3. Method (15) according to claim 1 or 2, **characterized in that** the mapping function (1) takes into account at least the change in the range of the particle beam.

4. Method (15) according to claim 3, **characterized in that** the change in the range of the particle beam caused by density changes is taken into account.

5. Method (15) according to any of the preceding claims, **characterized in that** the mapping function (1) depends in addition on an irradiation direction of the preferably moving particle beam.

6. Method (15) according to any of the preceding claims, **characterized in that** the dose administered to at least one target point volume (Vⱼ) is determined distinguishably at least in part for differing movement states (k) and/or for differing irradiation directions.

7. Method (15) according to any of the preceding claims, **characterized in that** the target point volumes (Vⱼ) are classified at least in part as target point volumes (Vⱼ) to be irradiated or as target point volumes (Vⱼ) not to be irradiated.

8. Method (15) according to any of the preceding claims, **characterized in that** a plurality of movement states (k) describing a typical movement sequence is used when creating the irradiation plan.

9. Method (15) according to any of the preceding claims, in particular according to claim 9, **characterized in that** a subselection of movement states (k) is used for creating the irradiation plan.

10. Method (15) according to any of the preceding claims, **characterized in that** a required dose and/or a tolerance dose is/are allocated to at least parts of the target point volumes (Vⱼ).

11. Method (15) according to one of the preceding claims, **characterized in that** a data matrix (1) is used as mapping function (1) or at least in some areas.

12. Method (15) according to one of the preceding claims, **characterized in that** an optimization in respect of a required dose distribution is carried out.

13. Method (15) according to any of the preceding claims, **characterized in that** during calculation of the irradiation plan at least two movement states (k) or at least two directions of irradiation are taken into account.

14. Device for creating an irradiation plan for irradiating a moving target volume area (11, 13) with a preferably moving particle beam, **characterized in that** it is designed and configured to implement a method (15) according to any of claims 1 to 13.

15. Device according to claim 14, **characterized in that** it has at least one program-controlled computing unit.

## Revendications

1. Procédé (15) pour créer un plan d'irradiation destiné à l'irradiation d'une zone de volume cible (11, 13) déplacée au moins partiellement, dans un objet (10) avec un faisceau de particules, sachant que le plan d'irradiation comprend des points d'irradiation (B) qui sont atteints par le faisceau de particules dans le cadre de l'irradiation et qui représentent respectivement un jeu de paramètres de commande pour le faisceau de particules, le procédé comprenant les étapes suivantes :
- Définition d'une pluralité d'états de déplacement (k) pour la zone de volume cible à irradier ;
- Détermination du volume à irradier pour chacun des états de déplacement (k) ;
- Détermination d'une pluralité de volumes de points cibles (Vⱼ) dans la zone de volume cible déplacée au moins partiellement ;
- Création d'une fonction de représentation (1) entre les points d'irradiation (Bᵢ) et les volumes de points cibles (Vⱼ), sachant que la fonction de représentation (1) contient des valeurs qui décrivent pour chacun de la pluralité des états de déplacement (k) la dose émise dans au moins un volume de points cibles (Vⱼ) si le i-ème point d'irradiation (Bᵢ) est atteint ;
- Création du plan d'irradiation en utilisant la fonction de représentation (1),
**caractérisée en ce qu'**un modèle d'irradiation essentiellement similaire est utilisé pour calculer le plan d'irradiation pour une pluralité de volumes de points cibles (Vⱼ) dans différents états de déplacement (k).

2. Procédé (15) selon la revendication 1, **caractérisé en ce que** le faisceau de particules est un faisceau de particules déplacé.

3. Procédé (15) selon la revendication 1 ou 2, **caractérisé en ce que** la fonction de représentation (1) tient compte au moins de la modification de portée du faisceau de particules.

4. Procédé (15) selon la revendication 3, **caractérisé en ce qu'**est prise en compte la modification de portée du faisceau de particules qui est due aux modifications de densité.

5. Procédé (15) selon une des revendications précédentes, **caractérisé en ce que** la fonction de représentation (1) dépend en plus d'un sens d'irradiation du faisceau de particules de préférence déplacé.

6. Procédé (15) selon une des revendications précédentes, **caractérisé en ce que** la dose est déterminée dans au moins un volume de points cibles (Vⱼ) au moins partiellement pour différents états de déplacement (k) et/ou pour différents sens d'irradiation, de manière à ce qu'ils puissent être distingués les uns des autres.

7. Procédé (15) selon une des revendications précédentes, **caractérisé en ce que** les volumes de points cibles (Vⱼ) sont classifiés au moins partiellement comme volumes de points cibles (Vⱼ) à irradier ou comme volumes de points cibles (Vⱼ) à ne pas irradier.

8. Procédé (15) selon une des revendications précédentes, **caractérisé en ce qu'**est utilisée, lors de la création du plan d'irradiation, une pluralité d'états de déplacement (k) qui décrivent un déroulement typique de déplacements.

9. Procédé (15) selon une des revendications précédentes, notamment selon la revendication 9, **caractérisé en ce qu'**une sous-sélection d'états de déplacement (k) est utilisée pour la création du plan d'irradiation.

10. Procédé (15) selon une des revendications précédentes, **caractérisé en ce qu'**une dose de consigne et/ou une dose de tolérance est attribuée à au moins des parties des volumes de points cibles (Vⱼ)

11. Procédé (15) selon une des revendications précédentes, **caractérisé en ce qu'**une matrice de données (1) est utilisée comme fonction de représentation (1) ou au moins partiellement.

12. Procédé (15) selon une des revendications précédentes, **caractérisé en ce qu'**a lieu une optimisation concernant une répartition de la dose de consigne.

13. Procédé (15) selon une des revendications précédentes, **caractérisé en ce que** lors du calcul du plan d'irradiation, au moins deux états de déplacement (k) ou au moins deux sens de l'émission d'irradiation sont pris en compte.

14. Dispositif pour créer un plan d'irradiation destiné à l'irradiation d'une zone de volume cible déplacée (11, 13) avec un faisceau de particules de préférence déplacé, **caractérisé en ce que** ledit dispositif est conçu et configuré de sorte à réaliser un procédé (15) selon une des revendications 1 à 13.

15. Dispositif selon la revendication 14, **caractérisé en ce que** celui-ci présente au moins une unité de calcul commandée par un programme.
